# EUROPEAN PATENT APPLICATION

(11) **EP 2 221 364 A1**
(43) Date of publication of application: **25.08.2010**
(21) Application number: 09305169.6
(22) Date of filing: 23.02.2009
(51) Int. Cl.: C12N 5/0783, A61P 37/06

(54) **Compositions for treating an allergic or asthmatic condition**

(71) Applicant: TXCell, 06560 Valbonne (FR)
(72) Inventor: Foussat, Arnaud, 06410 Biot (FR); Brun, Valérie, 06410 Biot (FR)

(57) **Abstract**

The present invention relates to compositions comprising human Tr1 cells directed to an allergen associated to an allergen or asthmatic provided said allergen is not a food allergen and use thereof for treating an allergic or asthmatic condition, excluding a food allergic condition.

## Description

### FIELD OF THE INVENTION

The present invention relates to the field of treatment of allergy and asthma. The invention relates in particular to methods for treating an allergic or asthmatic condition, using a medicament comprising human Tr1 cells directed against an allergic or asthmatic condition -associated antigen.

### BACKGROUND

Allergic diseases, including asthma, are defined as chronic inflammatory disorders originating from an aberrant immune response to innocuous antigens. Hence, the rapid and geographically localized nature of the increase in the incidence of allergic diseases indicates corresponding effects of recent changes in environment and lifestyle. One hypothesis based on the role of environment and lifestyle states that inappropriate or defective development of important immunological regulatory controls results from inadequate exposure to environmental microorganisms.

Allergic and atopic individuals have comparatively high levels of circulating IgE that is specific from common innocuous environmental allergens, such as house dust mites and grass pollens. Exposure to an allergen following allergic sensitization leads to cross-linking of allergen specific-IgE bound to the surface of mast cells and basophils, degranulation of these cells and release of histamine and other mediators that lead to the symptoms associated with early or acute allergic reactions, including wheezing and conjunctivitis.

Regarding the mechanism of said selective enhancement of allergen-specific IgE responses in allergic patients, Th2 cells play a major role. For example, the level of Th2-derived cytokines, such as IL-4, IL-5, IL-9 and IL-13, have been shown to be higher in allergic patients.

Current treatments for allergy and asthma are mainly based on the use of antihistamines, glucocorticoids, cortisone, dexamethasone, hydrocortisone, epinephrine (adrenaline), theophylline, cromolyn sodium, or β2-agonists. Anti-leukotrienes, such as Montelukast (Singulair) or Zafirlukast (Accolate), are FDA approved for treatment of allergic diseases.

Anti-cholinergics, decongestants, mast cell stabilizers, and other compounds thought to impair eosinophil chemotaxis, are also commonly used. These drugs help to alleviate the symptoms of allergy, and are imperative in the recovery of acute anaphylaxis, but play little role in chronic treatment of allergic disorders. In addition, said drugs may have some side effects. Finally, about one third of the patients do not achieve optimal disease control with these drugs.

Thus, there is a need for new strategies that have specific and long-lasting effects for the treatment of allergic and asthmatic condition.

In the present invention, the inventors aim to provide a specific treatment for allergy or asthma based on the use of Tr1 cells directed to a specific allergen-associated antigen.

### SUMMARY OF THE INVENTION

One object of the invention is a composition comprising at least one human Tr1 cell population directed against an allergen associated to an allergic or asthmatic condition, provided said allergen is not a food allergen.

According to one embodiment, said human Tr1 cell population is a human Tr1 clone population.

According to another embodiment, said allergen associated to an allergic or asthmatic condition is selected from the group comprising inhaled allergens, ingested allergens and contact allergens provided that it is not a food allergen.

According to another embodiment, said allergen associated to an allergic or asthmatic condition is selected in the group of allergens from house dust mites, fragments, variants and mixtures thereof.

According to another embodiment, said allergen associated to an allergic or asthmatic condition is selected in the group of allergens from pollens, fragments, variants and mixtures thereof.

According to another embodiment, said allergen associated to an allergic or asthmatic condition is selected in the group of allergens from dog, cat, rodents, fragments, variants and mixtures thereof.

Another object of the invention is a medicament or pharmaceutical composition comprising at least one human Tr1 cell population directed to an allergen associated to an allergic or asthmatic condition provided said allergen is not a food allergen.

According to one embodiment, said human Tr1 cell population is a human Tr1 clone population.

According to another embodiment, said human Tr1 cell population is directed to an allergen associated to an allergic or asthmatic condition selected from the group comprising inhaled allergens, ingested allergens and contact allergens.

According to another embodiment, said human Tr1 cell population is directed to allergens selected in the group of allergens from house dust mites, pollens, dog, cat, rodents, fragments, variants and mixtures thereof.

According to another embodiment, said medicament or pharmaceutical composition is for treating an allergic or asthmatic condition, excluding a food allergic condition.

According to one embodiment, said allergic or asthmatic condition is asthma, atopic dermatitis, allergic rhinitis, conjunctivitis, eczema and anaphylaxis.

According to another embodiment, said medicament or pharmaceutical composition is to be administrated to a subject of an effective amount and in combination with one or more therapeutic agents used for treating an allergic or asthmatic condition.

According to another embodiment, said medicament or pharmaceutical composition is for treating a subject predisposed with asthma.

Another object of the invention is a method for treating an allergic or asthmatic condition, excluding a food allergic condition, in a subject in need thereof, said process comprising the steps of:
- obtaining Tr1 cells directed to a selected allergen associated to an allergic or asthmatic condition provided said allergen is not a food allergen, said Tr1 cells being obtained from a blood sample of said subject,
- cloning said Tr1 cells directed to a selected allergen associated to an allergic or asthmatic condition,
- further expanding Tr1 clones obtained at the previous step,
- injecting Tr1 clones thus obtained in said subject, preferably by intravenous route.

### DETAILED DESCRIPTION OF THE INVENTION

### Definition

The term "Tr1 cells" as used herein refers to cells having the following phenotype at rest CD4+CD25-FoxP3- and capable of secreting high levels of IL-10 and significant levels TGF-β upon activation. Tr1 cells are characterized, in part, by their unique cytokine profile: they produce high levels of IL-10, significant levels of TGF-β and intermediate levels of IFN-γ, but little or no IL-4 or IL-2. The cytokine production is typically evaluated in cultures of cells after activation with polyclonal activators of T lymphocytes such as anti-CD3+ anti-CD28 antibodies or Interleukin-2, PMA + ionomycin. Alternatively, the cytokine production is evaluated in cultures of cells after activation with the specific T-cell antigen presented by antigen presenting cells. High levels of IL-10 correspond to at least about 500 pg/ml, typically greater than about 1, 2, 4, 6, 8, 10, 12, 14, 16, 18, or 20 thousand pg/ml or more. Significant levels of TGF-β correspond to at least about 100 pg/ml, typically greater than about 200, 300, 400, 600, 800, or 1000 pg/ml or more. Intermediate levels of IFN-γ correspond to concentrations comprised between 0 pg/ml and at least 400 pg/ml, typically greater than about 600, 800, 1000, 1200, 1400, 1600, 1800, or 2000 pg/ml or more. Little or no IL-4 or IL-2 corresponds to less than about 500 pg/ml, preferably less than about 250, 100, 75, or 50 pg/ml, or less.

The term "antigen" as used herein refers to a protein, or peptide to which the cells of this invention are being directed. In one embodiment, the term "antigen" may refer to a synthetically derived molecule, or a naturally derived molecule, which shares sequence homology with an antigen of interest, or structural homology with an antigen of interest, or a combination thereof. In one embodiment, the antigen may be a mimetope. A "fragment" of the antigen refers to any subset of the antigen, as a shorter peptide. A "variant" of the antigen refers to a molecule substantially similar to either the entire antigen or a fragment thereof. Variant antigens may be conveniently prepared by direct chemical synthesis of the variant peptide, using methods well-known in the art.

The term "subject" as used herein refers to a human being.

The term "effective amount" as used herein refers to an amount sufficient to cause a beneficial or desired clinical result (e.g. improvement in clinical condition).

The term "clone" or "clone population" as used herein refers to a population of differentiated cells being derived from a unique differentiated cell.

The term "treatment" or "treating" as used herein generally refers to a clinical intervention in an attempt to alter the natural course of the individual being treated, and may be performed during the course of clinical pathology. Desirable effects include, but are not limited to, alleviating symptoms, suppressing, diminishing or inhibiting any direct or indirect pathological consequences of the disease, lowering the rate of disease progression, ameliorating or palliating the disease state, and causing remission or improved prognosis. In the context of the invention, it refers to any improvement in the clinical symptoms of allergy or asthma, as well as any improvement in the well being of the patients.

The term "allergy" as used herein refers to any allergic condition such as anaphylaxis, eczema, rhinitis, conjunctivitis, the symptoms of which include hay fever, nasal blockage, and itchy and runny nose.

The term "asthma" as used herein refers to a chronic disease involving the respiratory system in which the airways occasionally constrict, become inflamed, and are lined with excessive amounts of mucus. This airway narrowing causes symptoms such as wheezing, shortness of breath, chest tightness, and coughing.

### The invention

Various methods for assessing allergy or asthma are known such as skin testing and blood testing.

For example, for assessing the presence of allergen-specific IgE antibodies, allergy skin testing is preferred over blood allergy tests because it is more sensitive and specific, simpler to use, and less expensive. Skin testing is also known as "puncture testing" and "prick testing" due to the series of tiny punctures or pricks made into the patient's skin. Small amounts of suspected allergens and/or their extracts (pollen, grass, mite proteins, peanut extract, etc.) are introduced to sites on the skin marked with pen or dye (the ink/dye should be carefully selected, lest it causes an allergic response itself). A small plastic or metal device is used to puncture or prick the skin. Sometimes, the allergens are injected "intradermally" into the patient's skin, with a needle and syringe. Common areas for testing include the inside forearm and the back. If the patient is allergic to the substance, then a visible inflammatory reaction will usually occur within 30 minutes. This response will range from slight reddening of the skin to a full-blown hive (called "wheal and flare") in more sensitive patients. Interpretation of the results of the skin prick test is normally done by allergists on a scale of severity, with +/- meaning borderline reactivity, and 4+ being a large reaction.

Various blood allergy testing methods are also available for detecting allergy to specific substances. This kind of testing measures a "total IgE level" - an estimate of IgE contained within the patient's serum. This can be determined through the use of radiometric and colormetric immunoassays. Radiometric assays include the radioallergosorbent test (RAST) method, which uses IgE-binding (anti-IgE) antibodies labeled with radioactive isotopes for quantifying the levels of IgE antibodies in the blood. Other newer methods use colorimetric or fluorometric technologies in the place of radioactive isotopes. Some "screening" test methods are intended to provide qualitative test results, giving a "yes" or "no" answer in patients with suspected allergic sensitization.

A low total IgE level is not adequate to rule out sensitization to commonly inhaled allergens. Statistical methods, such as ROC curves, predictive value calculations, and likelihood ratios have been used to examine the relationship of various testing methods to each other. These methods have shown that patients with a high total IgE level have a high probability of allergic sensitization, but further investigation with specific allergy tests for a carefully chosen allergen is often warranted.

All these methods allow the determination of the at least one allergen that is responsible for an allergic condition or an asthma condition.

The present invention aims to provide a method for treating at least one specific allergic or asthmatic condition, excluding food allergic condition, based on the use of at least one human Tr1 cell population directed against an allergen associated to said specific allergic or asthmatic condition.

The present invention relates to a method for treating at least one specific allergic or asthmatic condition, excluding food allergic condition, in a subject in need thereof, comprising the administration to said subject of a composition comprising human Tr1 cells directed against an allergen associated to said specific allergic or asthmatic condition.

According to the invention, the "human Tr1 cell population" corresponds to Tr1 cells as described here above in the definitions and does not include CD4+CD25+ regulatory T cells or FoxP3+ regulatory T cells (natural or conventional Treg), TGF-β secreting Th3 cells, or regulatory NKT cells.

According to the invention, "the allergens associated to said specific allergic or asthmatic condition, excluding food allergic condition" are selected in the group of inhaled allergen, ingested allergen excluding food allergen, and contact allergen.

Examples of inhaled allergens include, but are not limited to, allergens from:
- Astigmata : Acarus siro (Storage mite, Aca s 13), Blomia tropicalis (Mite, Blo t), Dermatophagoides farinae (American house dust mite, Der f), Dermatophagoides microceras (European house dust mite, Der p), Euroglyphus maynei (House dust mite, Eur m), Glycyphagus domesticus (Storage mite, Gly d 2), Lepidoglyphus destructor (Storage mite, Lep d), Tyrophagus putrescentiae (Storage mite, Tyr p);
- Blattaria: Blattella germanica (German cockroach, Bla g), Periplaneta americana (American cockroach, Per a)
- Coleoptera: Harmonia axyridis (Asian ladybeetle, Har a),
- Diptera: Aedes aegypti (Yellow fever mosquito, Aed a), Chironomus kiiensis (Midge, Chi k), Chironomus thummi thummi (Midge, Chi t), Forcipomyia taiwana (Biting midge, For t),
- Hemidiptera: Triatoma protracta (California kissing bug, Tria p),
- Hymenoptera: Apis cerana (Eastern hive bee, Api c), Apis dorsata (Giant honeybee, Api d), Apis mellifera (Honey bee, Api m), Bombus pennsylvanicus (Bumble bee, Bom p), Bombus terrestris (Bumble bee, Bom t), Dolichovespula arenaria (Yellow hornet, Dol a), Dolichovespula maculata (White face hornet, Dol m), Myrmecia pilosula (Australian jumper ant, Myr p), Polistes annularis (Wasp, Pol a), Polistes dominulus (Mediterranean paper wasp, Pol d), Polistes exclamans (Wasp, Pol e), Polistes fuscatus (Wasp, Pol f), Polistes gallicus (Wasp, Pol g), Polistes metricus (Wasp, Pol m), Polybia paulista (Wasp, Pol p), Polybia scutellaris (Wasp, Pol s), Solenopsis geminata (Tropical fire ant, Sol g), Solenopsis invicta (Red imported fire ant, Sol i), Solenopsis richteri (Black fire ant, Sol r), Solenopsis saevissima (Brazilian fire ant, Sol s), Vespa crabro (European hornet, Vesp c), Vespa mandarinia (Giant asian hornet, Vesp m), Vespula flavopilosa (Yellow jacket, Vesp f), Vespula germanica (Yellow jacket, Vesp g), Vespula maculifrons (Yellow jacket, ves m), Vespula pensylvanica (Yellow jacket, Vesp p), Vespula squamosa (Yellow jacket, Vesp s), Vespula vidua (Wasp, Ves vi), Vespula vulgaris (Yellow jacket, Ves v),
- Ixodida: Argas reflexus (Pigeon tick, Arg r),
- Lepidoptera: Thaumetopoea pityocampa (Pine processionary moth, Tha p),
- Siphonaptera: Ctenocephalides felis felis (Cat flea, Cte f),
- Carnivora: Canis familiaris (dog, Can f), Felis domesticus (cat, Fel d),
- Lagomorpha: Oryctolagus cuniculus (rabbit, Ory c),
- Perissodactlyla: Equus caballus (domestic horse, Equ c),
- Pleuronectiformes: Lepidorhombus whiffiagonis (Megrim, Whiff, Gallo, Lep w),
- Rodentia: Cavia porcellus (guinea pig, Cav p), Mus musculus (mouse, Mus m), Rattus norvegius (rat, Rat n),
- Coniferales: Chamaecyparis obtusa (Japanese cypress, Cha o), Cupressus arizonica (Cypress, Cup a), Cryptomeria japonica (Sugi, Cry j), Cupressus sempervirens (Common cypress, Cup s), Juniperus ashei (Mountain cedar, Jun a), Juniperus oxycedrus (Prickly juniper, Jun o), Juniperus sabinoides (Mountain cedar, Jun s), Juniperus virginiana (Eastern red cedar, Jun v),
- fragments or variants thereof.

Examples of ingested allergens excluding food allergens include, but are not limited to, allergens from Fungi Ascomycota:
- Dothideales: Alternaria alternata (Alternaria rot fungus, Alt a), Cladosporium cladosporioides (Cla c), Cladosporium herbarum (Cla h), Curvularia lunata (Cur 1),
- Eurotiales: Aspergillus flavus (Asp fl), Aspergillus fumigatus (Asp f), Aspergillus niger (Asp n), Aspergillus oryzae (Asp o), Penicillium brevicompactum (Pen b), Penicillium chrysogenum (Pen ch), Penicillium citrinum (Pen c), Penicillium oxalicum (Pen o),
- Hypocreales: Fusarium culmorum (Fus c),
- Onygenales : Trichophyton rubrum (Tri r), Trichophyton tonsurans (Tri t),
- Saccharomycetales: Candida albicans (Yeast, Cand a), Candida boidinii (Yeast, Cand b),
- Tuberculariales: Epicoccum purpurascens (Epi p), and Fungi Basidiomycota :

- Hymenomycetes : Coprinus comatus (Shaggy mane, Cop c), Psilocybe cubensis (Magic mushroom, Psi c),
- Urediniomycetes : Rhodotorula mucilaginosa (Yeast, Rho m),
- Ustilaginomycetes : Malassezia furfur (Pityriasis versicolor infect. Agent, Mala f), Malassezia sympodialis (Mala s), and antibiotics (such as Penicillins, Cephalosporins, Aminosides, Quinolones, Macrolides, Tetracyclins, Sulfamids ), drugs (such acetylsalicylic acid, vaccines, morphines and derivatives), vitamins such as vitamin K1,
- fragments or variants thereof.

The person skilled in the art will be able to find the sequences corresponding to each cited allergen. For example, said sequences or accession number may be found on the website www.allergen.org.

Examples of contact allergens include, but are not limited to, heavy metals (such as nickel, chrome, gold), latex, haptens such as halothane, hydralazine. The latter compounds, despite non their non protein based structure are able to modify self-proteins by direct integration in the protein structure leading to immunogenicity and induction of an allergic response. In this context, Tr1 regulatory lymphocytes can be produced in order to recognize specifically the modified self-proteins, responsible of allergic response induction.

As used herein, the term "allergen associated to said specific allergic or asthmatic condition, excluding food allergic condition" excludes food allergen such as allergen from milk, egg, peanut, tree nut (walnut, cashew, etc.), fish, shellfish, soy, wheat, and carrot, apple, pear, avocado, apricot, peach.

In a preferred embodiment, said human Tr1 cells are directed to allergens derived from pollens (Cup, Jun), house dust mites (Der, Gly, Tyr, Lep), dog, cat and rodents (Can, Fel, Mus, Rat).

Without whishing to be bound to a theory, the Applicant assume that the injected Tr1 cell population directed to an allergen associated to a specific allergic or asthmatic condition, excluding food allergic condition, would be activated in vivo by the antigen present in the subject and then would be able to control said allergic or asthmatic condition.

In one embodiment of the invention, human Tr1 cells may be obtained by
a) isolating a progenitor cell population from a subject,
b) obtaining a population of dendritic cells by culturing said progenitor cell population in the presence of IL-10,
c) contacting cells of step b) with a CD4+ T lymphocyte population isolated from said subject in the presence of an allergen associated to a specific allergic or asthmatic condition, excluding food allergic condition, to allow differentiation of CD4+ T cells directed to said antigen into the Tr1 cell population, and
d) recovering the Tr1 cell population from the step c).

In step b), IL-10 is present from 50 to 250 U/ml, preferably at 100 U/ml in the culture medium. Said method for obtaining Tr1 cells is described in Wakkach et al (Immunity 2003 May; 18(5):605-17).

Said method may also be carried out using Dexamethasone and Vitamin D3, or tolerogenised or immature DCs instead of the DCs of step b).

In another embodiment of the present invention, human Tr1 cells may be obtained by:
a) culturing a CD4+ T cell population directed to an allergen associated to a specific allergic or asthmatic condition, excluding food allergic condition, isolated from a subject in a media with an appropriate amount of IFN-α, and
b) recovering the Tr1 cell population.

IFN-α is preferably present in the media at 5 ng/ml. In the step a), the media may further comprise an appropriate amount of IL-10, preferably at 100 U/ml.

In step b), the Tr1 cell population is cultured in a media comprising IL-15 to allow proliferation, IL-15 being preferably at 5 ng/ml in the media. Said method for obtaining Tr1 cells is described in the patent US6746670.

In still another embodiment of the invention, human Tr1 cells may be obtained by:
a) in vitro activating a CD4+ T cell population in presence of an allergen associated to a specific allergic or asthmatic condition, excluding food allergic condition, presented by artificial antigen presenting cells, and
b) recovering an activated CD4+ T cells comprising at least 10% of Tr1 cells.

Preferably, the artificial antigen presenting cells express a HLA II system molecule and a human LFA-3 molecule and do not express the co-stimulation molecules B7-1, B7-2, B7-H1, CD40, CD23 and ICAM-1.

Said process, for obtaining Tr1 cells is described in the patent application WO021092793.

In still another embodiment of the invention, human Tr1 cells may be obtained by:
a) in vitro activating a CD4+ T cell population in presence of an allergen associated to a specific allergic or asthmatic condition, excluding food allergic condition and an appropriate amount of IL-10; and
b) recovering the Tr1 cell population.

Preferably, IL-10 is present in the media at 100 U/ml. Said method is described in Groux et al. (Nature 1997, 389(6652):737-42).

In still another embodiment of the invention, human Tr1 cells may be obtained by:
a) stimulating a leukocyte population or a peripheral blood mononuclear cell (PBMC) population with an allergen associated to a specific allergic or asthmatic condition, excluding food allergic condition,
b) Recovering the antigen-specific Tr1 cell population from the stimulated population,
c) Optionally expanding said antigen-specific Tr1 cell population.

Leukocytes encompass several types of cells, which are characterized by their importance, their distribution, their number, their lifetime and their potentiality. These types are the following : the polynuclear or granular leukocytes, among which one finds the eosinophilic, the neutrophilic and the basophilic leukocytes, and the mononuclear cells, or peripheral blood mononuclear cells (PBMCs), which are large white blood cells and consist in the major cell types of the immune system (lymphocytes and monocytes). The leukocytes or the PBMCs can be separated from the peripheral blood by any method known to those skilled in the art. Advantageously, for the separation of the PBMCs, centrifugation may be used, preferably density gradient centrifugation, preferably discontinuous density gradient centrifugation. An alternative is the use of specific monoclonal antibodies. In certain embodiments PBMC are typically isolated from the whole blood product by means of Ficoll-Hypaque, using standard procedures. In other embodiments the PBMCs are recovered by means of leukapheresis.

Said method is described in the patent application WO2007/010406.

In still another embodiment, human Tr1 cells may be obtained by:
a) culturing a leukocyte population or a peripheral blood mononuclear cell (PBMC) population with mesenchymal stem cells in the presence of an allergen associated to a specific allergic or asthmatic condition, excluding food allergic condition,
b) recovering the Tr1 cell population.

Said method can also be carried out with naïve or memory T cells instead of PBMC or leukocytes.

The Tr1 cell population thus obtained may further be expanded by culture in presence of cytokines such as Interleukin-2 and Interleukin-4. Alternatively, Interleukin-15 and

Interleukin-13 could also be used in Tr1 cell expansion cultures.

In the methods described above, human Tr1 cells can be characterized by the identification method described in WO2005/000344. Said identification method of Tr1 cells is based on the detection of the simultaneous presence of expression products of genes coding CD4 molecule and molecules from the group comprising CD18 and/or CD11a, and CD49b. Tr1 cells can be identified and/or purified by Elisa, flow cytometry, or immunoaffinity methods with antibodies directed against said markers.

Tr1 cells can also be enriched by positive selection or negative selection using flow cytometry or magnetic beads. Such methods are also described in WO2005/000344.

In another embodiment of the present invention, the Tr1 cells directed to an allergen associated to a specific allergic or asthmatic condition, excluding food allergic condition may be expanded by the in vitro method described in WO2006/108882. Said method comprises:
a) cultivating at a temperature T1 inferior to 35°C, in a culture medium Mf, feeder cells such as insect feeder cells, said temperature T1 allowing the proliferation of feeder cells and said feeder cells expressing factors which interact with the following cell surface proteins:
   - the CD3/TCR complex,
   - the CD28 protein,
   - the IL-2 receptor,
   - the CD2 protein,
   - the IL-4 receptor,
b) contacting the feeder cells obtained in step a) cleared or not of their culture medium Mf, with the Tr1 cell population contained in the culture medium Mp, wherein said culture medium Mp does not initially contain the factors cited in step a), in order to obtain a mixture containing the Tr1 cell population, the feeder cells and the culture medium Mp,
c) cultivating the mixture obtained at step b) at a temperature T2 which is at least 35°C, said temperature being chosen such that the Tr1 cell population proliferates and the feeder cells do not proliferate,
d) recovering the Tr1 cell population such expanded.

Examples of factors which interact with the above mentioned cell surface proteins include:
- a modified anti-CD3 antibody, wherein the anti-CD3 intracytoplasmic domain of the CD3 heavy chain is replaced with a transmembrane domain,
- the CD80 or CD86 protein,
- the IL-2 secreted by the feeder cells,
- the CD58 protein,
- an interleukin selected from the group comprising IL-4 and IL-13.

In a preferred embodiment of the present invention, said Tr1 cells directed to an allergen associated to a specific allergic or asthmatic condition, excluding food allergic condition may be cloned by using conventional methods for cloning T cells.

In preferred embodiment of the present invention, said composition comprising at least one human Tr1 cell population directed against an allergen associated to a specific allergic or asthmatic condition, excluding food allergic condition, or at least one clone of human Tr1 cell directed against an allergen associated to a specific allergic or asthmatic condition, excluding food allergic condition, may be frozen to be stored.

In a preferred embodiment of the present invention, said allergen associated to a specific allergic or asthmatic condition, excluding food allergic condition is selected from the group comprising allergens derived from pollens (Cup, Jun), house dust mites (Der, Gly, Tyr, Lep), dog, cat and rodents (Can, Fel, Mus, Rat), fragments or variants thereof.

Preferably, said allergen associated to a specific allergic or asthmatic condition, excluding food allergic condition, is selected from the group of allergens derived from pollens (Cup, Jun), fragments or variants thereof.

Preferably, said allergen associated to a specific allergic or asthmatic condition, excluding food allergic condition, is selected from the group of allergens derived from house dust mites (Der, Gly, Tyr, Lep) house dust mites (Der, Gly, Tyr, Lep), fragments or variants thereof.

Preferably, said allergen associated to a specific allergic or asthmatic condition, excluding food allergic condition, is selected from the group of allergens derived from dog, cat and rodents (Can, Fel, Mus, Rat), fragments or variants thereof.

The term "variant" of an allergen associated to a specific allergic or asthmatic condition, excluding food allergic condition, refers herein to an antigen that is almost identical to the natural antigen and which shares the same biological activity. The minimal difference between the natural antigen and its variant may lie for example in an amino-acid substitution, deletion, and/or addition. Such variants may contain for example conservative amino acid substitutions in which amino acid residues are replaced with amino acid residues having a similar side chain. Families of amino acid residues having similar side chains have been defined in the art, including basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine).

Another object of the present invention is to provide a medicament comprising at least one human Tr1 cell population directed to an allergen associated to a specific allergic or asthmatic condition, excluding food allergic condition.

The present invention also intends to provide a pharmaceutical composition comprising at least one human Tr1 cell population directed against an allergen associated to a specific allergic or asthmatic condition, excluding food allergic condition in combination with one or more pharmaceutically acceptable carrier.

According to a preferred embodiment, said human Tr1 cell population is a human Tr1 clone population.

According to a preferred embodiment, said allergen associated to a specific allergic or asthmatic condition, excluding food allergic condition, is selected in the group of allergens derived from pollens (Cup, Jun), house dust mites (Der, Gly, Tyr, Lep), dog, cat and rodents (Can, Fel, Mus, Rat), fragments, variants and mixtures thereof.

According to a more preferred embodiment, the medicament or the pharmaceutical composition of the invention comprises at least one human Tr1 cell population or clone directed against an allergen associated to a specific allergic or asthmatic condition, excluding food allergic condition selected in the group of allergens derived from pollens (Cup, Jun), house dust mites (Der, Gly, Tyr, Lep), dog, cat and rodents (Can, Fel, Mus, Rat), fragments, variants and mixtures thereof.

The pharmaceutically acceptable carriers useful herein are conventional. Remington's Pharmaceutical Sciences 16th edition, Osol, A. Ed. (1980) describes composition and formulations suitable for pharmaceutical delivery of the composition of the present invention. In general, the nature of the carrier will depend on the mode of administration being employed. For instance, parenteral formulations usually comprise injectable fluids that include pharmaceutically and physiologically acceptable fluids such as water, physiological saline, balanced salt solutions, aqueous dextrose, sesame oil, glycerol, ethanol, combinations thereof, or the like, as vehicle. The carrier and composition can be sterile, and the formulation suits the mode of administration. In addition to biological neutral carriers, pharmaceutical compositions to be administrated can contain minor amounts of non toxic auxiliary substances, such as wetting or emulsifying agents, preservatives, and pH buffering agents and the like, for example sodium acetate or sorbitan monolaurate. The composition can be a liquid solution, suspension, emulsion.

The invention relates to a pharmaceutical composition or medicament comprising at least one human Tr1 cell population directed against an allergen associated to a specific allergic or asthmatic condition, excluding food allergic condition, for treating said allergic or asthmatic condition.

The invention relates to a pharmaceutical composition or medicament comprising at least one human Tr1 cell population directed against an allergen associated to a specific allergic or asthmatic condition, excluding food allergic condition, for use in treating said allergic or asthmatic condition.

The invention relates to the use of a composition comprising at least one human Tr1 cell population directed against an allergen associated to a specific allergic or asthmatic condition, excluding food allergic condition, for the preparation of a medicament or a pharmaceutical composition for treating said allergic or asthmatic condition.

Said allergic or asthmatic condition includes, but is not limited to, asthma, atopic dermatitis, allergic rhinitis, conjunctivitis, eczema and anaphylaxis.

According to a preferred embodiment, said human Tr1 cell population is a human Tr1 clone population.

According to a preferred embodiment, said one human Tr1 cell population or clone is directed against an allergen associated to a specific allergic or asthmatic condition, excluding food allergic condition, selected in the group of allergens derived from pollens (Cup, Jun), house dust mites (Der, Gly, Tyr, Lep), dog, cat and rodents (Can, Fel, Mus, Rat), fragments, variants and mixtures thereof.

In one embodiment, the present invention relates to the use of a composition as described here above for the preparation of a medicament or a pharmaceutical composition for treating asthma.

In one embodiment, the present invention relates to the use of a composition as described here above for the preparation of a medicament or a pharmaceutical composition for treating eczema

In one embodiment, the present invention relates to the use of a composition as described here above for the preparation of a medicament or a pharmaceutical composition for treating anaphylaxis.

In another embodiment, the present invention relates to the use of a composition as described here above for the preparation of a medicament or a pharmaceutical composition for treating atopic dermatitis.

In another embodiment, the present invention relates to the use of a composition as described here above for the preparation of a medicament or a pharmaceutical composition for treating allergic rhinitis.

In another embodiment, the present invention relates to the use of a composition as described here above for the preparation of a medicament or a pharmaceutical composition for treating conjunctivitis.

An object of the present invention is also a method for treating allergic or asthmatic condition, excluding a food allergic condition, in a subject in need thereof, comprising administering to said subject an effective amount of a medicament as described here above or a pharmaceutical composition as described here above.

Said allergic or asthmatic conditions, excluding a food allergic condition, are preferably selected in the group of asthma, atopic dermatitis, allergic rhinitis, conjunctivitis, eczema and anaphylaxis.

The composition may be formulated for parenteral, intramuscular, intravenous, intraperitoneal, injection, intranasal inhalation, lung inhalation, intradermal, intrathecal.

Preferably, the medicament or pharmaceutical composition of the invention may be administrated by intranasal inhalation, lung inhalation, intraperitoneal or intravenous injection, or by direct injection into the lymph nodes of the patient, preferably by intravenous injection.

The amount of Tr1 cells directed an allergen associated to a specific allergic or asthmatic condition, excluding food allergic condition, effective in the treatment of an allergic or asthmatic condition will depend on the nature of the inflammation, and can be determined by standard clinical techniques. The precise dose to be employed in the formulation will also depend on the route of administration, and the seriousness of the disease or disorder, and should be decided according to the judgment of the practitioner and each individual's circumstances. Effective doses can be extrapolated from dose-response curves derived from in vitro or animal model test systems.

In one embodiment of the present invention, 10⁴/kg to 10⁹/kg cells are administrated to the subject. Preferably 10⁶/kg to 10⁸/kg cells and more preferably about 10⁸/kg cells are administrated to the subject.

In one embodiment of the invention, the subject is administrated with the medicament at the time when flare-up are demonstrated by a decline in the clinical status of the subject.

In one embodiment of the invention, the subject is administrated once with the medicament or the pharmaceutical composition of the present invention.

In a second embodiment of the invention, the subject is administrated once a month with the medicament or the pharmaceutical composition of the present invention.

In a third embodiment of the invention, the subject is administrated once a quarter with the medicament or the pharmaceutical composition of the present invention.

In a fourth embodiment of the invention, the subject is administrated once to twice a year with the medicament or the pharmaceutical composition of the present invention.

In another embodiment of the present invention, the medicament or pharmaceutical composition to be administered to a subject in need thereof comprises human Tr1 cells autologous to the cells of said subject.

This means that Tr1 cells will be administrated to the subject they come from or that precursors used for the production of Tr1 cells come from the subject the Tr1 cells will be administrated to.

The present invention relates also to a method for treating an allergic or asthmatic condition in a subject in need thereof, said process comprising the steps of:
- collecting a blood sample of said subject,
- obtaining Tr1 cells directed to a selected allergen associated to a specific allergic or asthmatic condition, excluding food allergic condition,
- cloning said Tr1 cells directed to a selected allergen associated to a specific allergic or asthmatic condition, excluding food allergic condition,
- further expanding Tr1 clones obtained at the previous step,
- injecting Tr1 clones thus obtained in said subject, preferably by intravenous route.

Preferably, cloning and expansion of Tr1 clones directed to a selected allergen associated to a specific allergic or asthmatic condition, excluding food allergic condition, is carried out with the following method:
a) cultivating at a temperature T1 inferior to 35°C, in a culture medium Mf, feeder cells such as insect feeder cells, said temperature T1 allowing the proliferation of feeder cells and said feeder cells expressing factors which interact with the following cell surface proteins:
   - the CD3/TCR complex,
   - the CD28 protein,
   - the IL-2 receptor,
   - the CD2 protein,
   - the IL-4 receptor,
b) contacting the feeder cells obtained in step a) cleared or not of their culture medium Mf, with the Tr1 cell population contained in the culture medium Mp, wherein said culture medium Mp does not initially contain the factors cited in step a), in order to obtain a mixture containing the Tr1 cell population, the feeder cells and the culture medium Mp,
c) cultivating the mixture obtained at step b) at a temperature T2 which is at least 35°C, said temperature being chosen such that the Tr1 cell population proliferates and the feeder cells do not proliferate,
d) recovering the Tr1 cell population such expanded.

Examples of factors which interact with the above mentioned cell surface proteins include:
- a modified anti-CD3 antibody, wherein the anti-CD3 intracytoplasmic domain of the CD3 heavy chain is replaced with a transmembrane domain,
- the CD80 or CD86 protein,
- the IL-2 secreted by the feeder cells,
- the CD58 protein,
- an interleukin selected from the group comprising IL-4 and IL-13.

In another embodiment of the invention, the method for treating an allergic or asthmatic condition, excepting a food allergic condition, in a subject in need thereof comprises the administration to said subject of an effective amount of the medicament or the pharmaceutical composition of the invention in combination with one or more therapeutic agents used for treating an allergic or asthmatic condition.

The invention relates to the use of the pharmaceutical composition or medicament of the invention, wherein the administration to said subject of an effective amount of the medicament or the pharmaceutical composition of the invention is in combination with one or more therapeutic agents used for treating an allergic or asthmatic condition.

Examples of therapeutic agents used for treating an allergic or asthmatic condition are the following: antihistamines, glucocorticoids (such as ciclesonide, beclomethasone, budesonide, flunisolide, fluticasone, mometasone, and triamcinolone), cortisone, dexamethasone, hydrocortisone, epinephrine (adrenaline), theophylline, cromolyn sodium, β2-agonists, anti-leukotrienes, (such as montelukast, zafirlukast, pranlukast, and zileuton), anti-cholinergics/antimuscarinics (such as ipratropium, oxitropium, and tiotropium), decongestants, mast cell stabilizers (such as cromoglicate (cromolyn), and nedocromil), methylxanthines (such as theophylline and aminophylline), compounds thought to impair eosinophil chemotaxis and IgE blockers (such as Omalizumab).

In another embodiment, the present invention also relates to a method of treatment of an allergic or asthmatic condition, excluding a food allergic condition, in which the medicament or the pharmaceutical composition of the invention is to be administrated to a subject in need thereof, wherein the subject is predisposed with asthma.

Asthma is caused by a complex interaction of environmental and genetic factors. These factors can also influence how severe a person's asthma is and how well they respond to medication. As with other complex diseases, many environmental and genetic factors have been suggested as causes of asthma. Over 100 genes have been associated with asthma in at least one genetic association study. Through the end of 2005, 25 genes had been associated with asthma in six or more separate populations. Many of these genes are related to the immune system or to modulating inflammation. Research also suggests that some genetic variants may only cause asthma when they are combined with specific environmental exposures, and otherwise may not be risk factors for asthma. Some of the genes associated with asthma are: GSTM1, IL10, CTLA-4, SPINK5, LTC4S, LTAG, RPA, NODI, CC16, GSTP1, STAT6, NOS1, CCL5, TBX, A2R, TGFB1, IL4, IL13, CD14, ADRB2 (β-2 adrenergic receptor), HLA-DRB1, HLA-DQB1, TNF, FCER1B, IL4R, ADAM33.

The present invention relates to the use of the pharmaceutical composition or medicament of the invention, wherein said subject does not respond adequately to, or is unlikely to respond adequately to, one or more therapeutic agent in the group of antihistamines, glucocorticoids, cortisone, dexamethasone, hydrocortisone, epinephrine (adrenaline), theophylline, cromolyn sodium, β2-agonists, anti-leukotrienes, anti-cholinergics/antimuscarinics, decongestants, mast cell stabilizers, methylxanthines. "Inadequate response", "does not respond adequately to", or "unlikely to respond adequately" refer to an actual or probable response by a subject which indicates that the therapy has been, or is likely to be, ineffective, toxic, or poorly tolerated insofar as the subject is concerned.

### BRIEF DESCRIPTION OF THE DRAWINGS

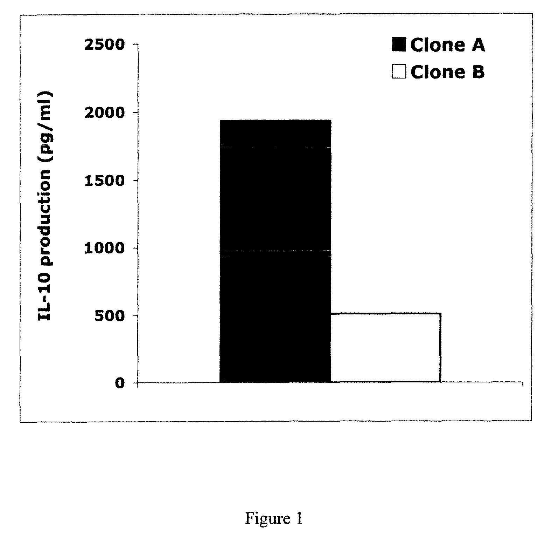

**Figure 1****:** IL-10 production of Tr1 clones after specific activation with Derp-1 allergen.
   Figure 1 describes the specific increase of the IL-10 production by T-cell clones in the presence of the specific allergen Der-1. Clones were activated with or without Derp-1 allergen in the presence of irradiated autologous antigen presenting cells. After 48 hours, the IL-10 production was measured by ELISA.
**Figure 2****:** Cytokine secretion profile of anti - Derp-1 allergen Tr1 clones
   IL-10, IL-4 and IFNγ secretion of Derp-1 allergen specific Tr1 cell clones were measured in 48 hours supernatant of anti-CD3 + anti-CD28 monoclonal antibodies activated cells.
**Figure 3****:** In vitro suppressive activity of anti - Der- allergen Tr1 clones
   The suppressive activity of Derp-1 allergen Tr1 clones was evaluated in coculture experiments with autologous CD4+ T lymphocytes. Cell populations were co-cultured during 3 days using anti-CD3 + anti-CD28 monoclonal antibodies. Then, cell proliferation of the autologous CD4+ T cells was assessed in the absence or presence of graded quantities of Tr1 cells. Results show that the addition of Tr1 cells to CD4+ T lymphocytes massively inhibits T-cell proliferation.

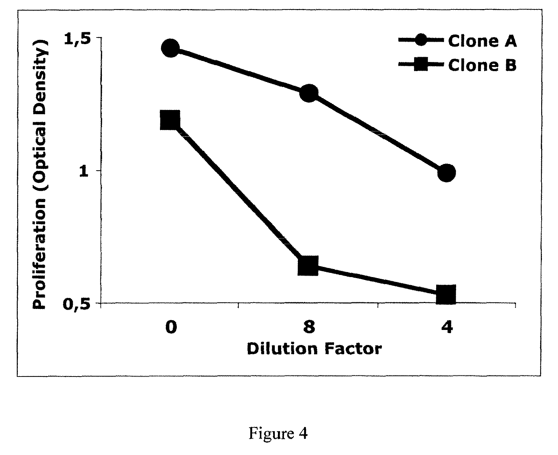

**Figure 4****:** Suppressive activity of anti - Derp-1 allergen Tr1 clones culture supernatants.
   CD4+ T lymphocytes were cultured during 3 days using anti-CD3 + anti-CD28 monoclonal antibodies. Then, cell proliferation of the CD4+ T cells was assessed in the presence of Tr1 cells culture supernatants. Results show that the addition of Tr1 cells culture supernatants to CD4+ T lymphocytes massively inhibits T-cell proliferation.

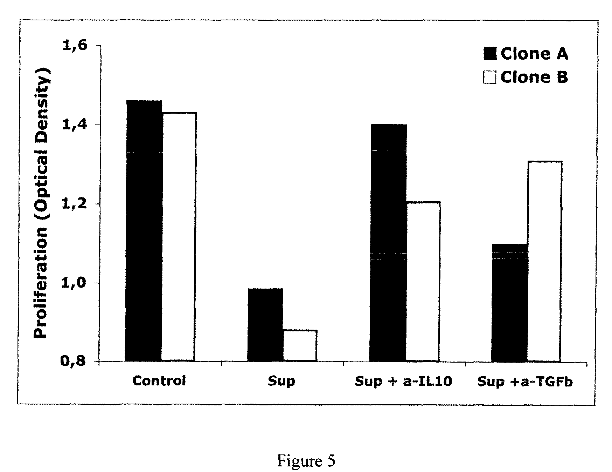

**Figure 5****:** Suppressive activity of anti - Derp-1 allergen Tr1 clones culture supernatants is inhibited by monoclonal antibodies anti-IL-10 and anti-TGF-β.

### EXAMPLES

In the following description, all experiments for which no detailed protocol is given are performed according to standard protocol.

The following examples are included to demonstrate preferred embodiments of the invention. It should be appreciated by those of skill in the art that the techniques disclosed in the below examples represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

### EXPERIMENTAL PROCEDURES

### TR1 CELL ISOLATION

Blood samples from healthy patients were collected and white blood cells were separated using gradient density centrifugation. Cells were then cultured in the presence of Derp-1 allergen (SEQ ID NO: 1) in order to induce the specific proliferation of Tr1 cells directed against this antigen. After 13 days of culture, cell populations were cloned by limiting dilution method. Clones were then assessed for their specificity to Derp-1 allergen and for characteristic Tr1 cytokine production profile.

### CYTOKINE ASSAYS

For the determination of antigen specificity, sandwich ELISAs were performed on 48 hours supernatants of T-cell clones stimulated in the presence of antigen presenting cells (4.10⁵) and in the presence or absence of the specific antigen (Derp-1 allergen). For the determination of the cytokine production profile, Derp-1 allergen Tr1 cell clones were stimulated with anti-CD3 + anti-CD28 monoclonal antibodies and the supernatants were harvested after 48 hours. ELISAs were performed using anti-IL-4 (11B11), anti-IL-10 (2A5), anti-IFN-γ (XGM1.2), biotin anti-IL-4 (24G2), anti-IL-10 (SXC1), anti-IFN-γ (R4-6A2) (Pharmingen Becton Dickinson).

### SUPPRESSION STUDIES

For suppression studies in co-culture, graded quantities of Derp-1 allergen specific Tr1 clones were co-cultured with autologous CD4+ T lymphocytes. Co-cultures were stimulated with anti-CD3 + anti-CD28 monoclonal antibodies coated beads. After 3 days, total cell proliferation was assessed using the WST-1 proliferation kit from Roche (Roche Applied. Science, Basel, Switzerland). Suppression of autologous CD4+ T-cell proliferation was also evaluated using diluted supernatants of Tr1 cells activated with anti-CD3 + anti-CD28 monoclonal antibodies coated beads during 48 hours. Anti-IL-10 and anti-TGFbeta monoclonal antibodies were purchased from R&D systems and were used at 10µg/ml.

### RESULTS

Figure 1 shows the IL-10 production of two distinct Tr1 cell populations specific for Derp-1 allergen in the presence or absence of the antigen. Results show that Derp-1 allergen stimulation induces an increase in the production of IL-10. These results demonstrate the specificity of the cell populations toward Derp-1 allergen.

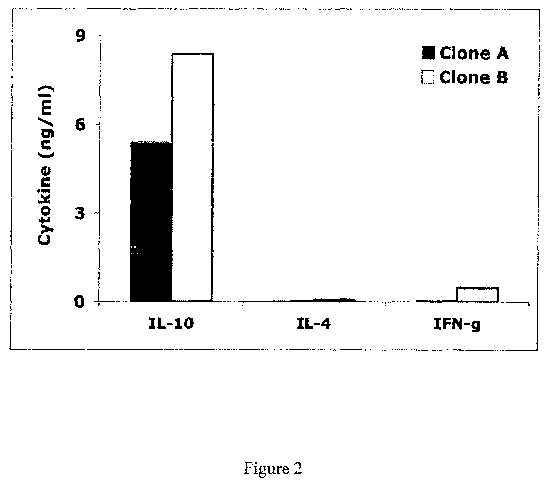

To further determine the cytokine secretion profile of these Tr1 cell populations specific for Derp-1 allergen, cells were stimulated in the presence of anti-CD3+anti-CD28 monoclonal antibodies. ELISAs were performed on 48h supernatants to measure IL-4, IL-10 and IFNγ production. Figure 2 shows that the cytokine secretion profile observed for the latter Derp-1 allergen specific populations corresponds to a Tr1 cytokine secretion profile, i.e. high production of IL-10, low production of IFNγ and no production of IL-4.

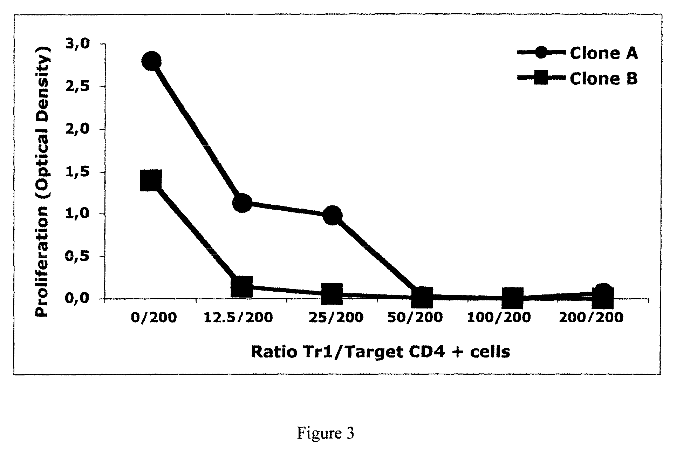

Suppressive activity of these Derp-1 allergen Tr1 populations was then assessed. Tr1 cells were co-cultivated with autologous CD4+ T cells in the presence of anti-CD3+anti-CD28 monoclonal antibodies. After 3 days of stimulation, cell proliferation was measured. Figure 3 shows the results for the two Tr1 populations and confirms the suppressive activity of these cells.

Suppressive activity of these anti-Derp-1 Tr1 clones was also found in culture supernatants (sup) as shown in Figure 4.

Figure 5 shows that this suppressive activity is mediated by the secretion of IL-10 and TGF-β.

## Claims

1. Composition comprising at least one human Tr1 cell population directed to an allergen associated to an allergic or asthmatic condition, provided said allergen is not a food allergen.

2. A composition according to claim **1,** wherein said human Tr1 cell population is a human Tr1 clone population.

3. A composition according to anyone of claim **1** or **2,** wherein said allergen associated to an allergic or asthmatic condition is selected from the group comprising inhaled allergens, ingested allergens and contact allergens provided that it is not food allergen.

4. A composition according to claim **3,** wherein said allergen associated to an allergic or asthmatic condition is selected in the group of allergens from house dust mites, fragments, variants and mixtures thereof.

5. A composition according to claim **3,** wherein said allergen associated to an allergic or asthmatic condition is selected in the group of allergens from pollens, fragments, variants and mixtures thereof.

6. A composition according to claim **3,** wherein said allergen associated to an allergic or asthmatic condition is selected in the group of allergens from dog, cat, rodents, fragments, variants and mixtures thereof.

7. Medicament or pharmaceutical composition comprising at least one human Tr1 cell population directed to an allergen associated to an allergic or asthmatic condition provided said allergen is not a food allergen.

8. Medicament or pharmaceutical composition according to claim **7,** wherein said human Tr1 cell population is a human Tr1 clone population.

9. Medicament or pharmaceutical composition according to any one of claim **7** to **8,** wherein said human Tr1 cell is directed to an allergen associated to an allergic or asthmatic condition selected from the group comprising inhaled allergens, ingested allergens and contact allergens.

10. Medicament or pharmaceutical composition according to any one of claim **7** to **9,** wherein said human Tr1 cell is directed to allergens selected in the group of allergens from house dust mites, pollens, dog, cat, rodents, fragments, variants and mixtures thereof.

11. Medicament or pharmaceutical composition according to anyone of claims **7** to **10** for treating an allergic or asthmatic condition, excluding a food allergic condition.

12. Medicament or pharmaceutical composition according to claim **11** for treating asthma, atopic dermatitis, allergic rhinitis, conjunctivitis, eczema and anaphylaxis.

13. Medicament or pharmaceutical composition according to anyone of claim **11** to **12,** wherein the administration to a subject of an effective amount of the medicament or the pharmaceutical composition of the invention is in combination with one or more therapeutic agents used for treating an allergic or asthmatic condition.

14. Medicament or pharmaceutical composition according to anyone of claim **11** to **13,** for treating a subject predisposed with asthma.

15. Method for treating an allergic or asthmatic condition, excluding a food allergic condition, in a subject in need thereof, said process comprising the steps of:
- obtaining Tr1 cells directed to a selected allergen associated to an allergic or asthmatic condition provided said allergen is not a food allergen, said Tr1 cells being obtained from a blood sample of said subject,
- cloning said Tr1 cells directed to a selected allergen associated to an allergic or asthmatic condition,
- further expanding Tr1 clones obtained at the previous step,
- injecting Tr1 clones thus obtained in said subject, preferably by intravenous route.


SEQUENCE LISTING

<110>  TXCELL
       Foussat, Arnaud

<120>  COMPOSITIONS FOR TREATING AN ALLERGIC OR ASTHMATIC CONDITION

<130>  BEP090126

<160>  1

<170>  PatentIn version 3.3

<210>  1
<211>  263
<212>  PRT
<213>  Dermatophagoides microceras

<400>  1

Leu Ser Val Val Tyr Ala Tyr Pro Ser Glu Ile Arg Thr Phe Glu Glu
1               5                   10                  15

Phe Lys Lys Ala Phe Asn Lys His Tyr Val Thr Pro Glu Ala Glu Gln
            20                  25                  30

Glu Ala Arg Gln Asn Phe Leu Ala Ser Leu Glu His Ile Glu Lys Ala
        35                  40                  45

Gly Lys Gly Arg Ile Asn Gln Phe Ser Asp Met Ser Leu Glu Glu Phe
    50                  55                  60

Lys Asn Gln Tyr Leu Met Ser Asp Gln Ala Tyr Glu Ala Leu Lys Lys
65                  70                  75                  80

Glu Phe Asp Leu Asp Ala Gly Ala Gln Ala Cys Gln Ile Gly Ala Val
                85                  90                  95

Asn Ile Pro Asn Glu Ile Asp Leu Arg Ala Leu Gly Tyr Val Thr Lys
            100                 105                 110

Ile Lys Asn Gln Val Ala Cys Gly Ser Cys Trp Ala Phe Ser Gly Val
        115                 120                 125

Ala Thr Val Glu Ser Asn Tyr Leu Ser Tyr Asp Asn Val Ser Leu Asp
    130                 135                 140

Leu Ser Glu Gln Glu Leu Val Asp Cys Ala Ser Gln His Gly Cys Gly
145                 150                 155                 160

Gly Asp Thr Val Leu Asn Gly Leu Arg Tyr Ile Gln Lys Asn Gly Val
                165                 170                 175

Val Glu Glu Gln Ser Tyr Pro Tyr Lys Ala Arg Glu Gly Arg Cys Gln
            180                 185                 190

Arg Pro Asn Ala Lys Arg Tyr Gly Ile Lys Asp Leu Cys Gln Ile Tyr
        195                 200                 205

Pro Pro Asn Gly Asp Lys Ile Arg Thr Tyr Leu Ala Thr Lys Gln Ala
    210                 215                 220

Ala Leu Ser Val Ile Ile Gly Ile Arg Asp Leu Asp Ser Phe Arg His
225                 230                 235                 240

Tyr Asp Gly Arg Thr Ile Leu Gln Ser Asp Asn Gly Gly Lys Arg Asn
                245                 250                 255

Phe His Ala Ile Asn Ile Val
            260